# EUROPEAN PATENT APPLICATION

(11) **EP 1 815 854 A1**
(43) Date of publication of application: **08.08.2007**
(21) Application number: 07008091.6
(22) Date of filing: 01.09.2003
(51) Int. Cl.: A61K 31/35, A61P 25/14

(54) **Treatment of dyskenesia**

(30) Priority: 17.09.2002 US 411597 P; 04.04.2003 GB 0307824
(62) Divisional of application: 03748249.4
(71) Applicant: Motac Neuroscience Limited, Manchester M15 6WE (GB)
(72) Inventor: Crossman, Alan, Manchester M13 9PT (GB); Silverdale, Montague, Fulwood, Preston PR2 9HT (GB); Hill, Michael, Manchester M15 6SE (GB); Brotchie, Jonathan, Toronto, Ontario M5T 2S8 (CA)
(74) Representative: Banford, Paul Clifford

(57) **Abstract**

The present invention relates to the treatment of dyskinesia with compounds of General Formula (I). The compound may be topiramate or a derivative thereof. The dyskinesia may be associated with a basil ganglia-related movement disorder such as parkinsonism and may also arise as a side-effect of other therapeutic agents (e.g. L-DOPA).

## Description

The present invention relates to the treatment of dyskinesias.

Dyskinesias are abnormal involuntary movement disorders. The abnormal movements may manifest as chorea (involuntary, rapid, irregular, jerky movements that may affect the face, arms, legs, or trunk), ballism (involuntary movements similar to chorea but of a more violent and forceful nature), dystonia (sustained muscle contractions, usually producing twisting and repetitive movements or abnormal postures or positions) or athetosis (repetitive involuntary, slow, sinuous, writhing movements, which are especially severe in the hands).

Movement and other disorders due to dysfunction of the basal ganglia and related brain structures are of major socio-economic importance. Such disorders can occur as a consequence of inherited or acquired disease, idiopathic neurodegeneration or they may be iatrogenic. The spectrum of disorders is very diverse, ranging from those associated with poverty of movement (akinesia, hypokinesia, bradykinesia) and hypertonia (e.g. Parkinson's disease, some forms of dystonia) to the involuntary movement disorders (hyperkinesias or dyskinesias e.g. Huntington's disease, levodopa-induced dyskinesia, ballism, and some forms of dystonia).

Parkinsonism is a well known movement disorder comprising a syndrome characterised by slowness of movement (bradykinesia), rigidity and/or tremor. Parkinsonian symptoms are seen in a variety of conditions, most commonly in idiopathic parkinsonism (*i.e.,* Parkinson's disease) but also following treatment of schizophrenia, exposure to toxins/drugs and head injury. In Parkinson's disease the primary pathology is degeneration of dopaminergic neurons of the substantia nigra, pars compacta.

The most widely used symptomatic treatments for parkinsonism use dopamine-replacing agents (e.g. L-DOPA and dopamine receptor agonists). One common way in which dyskinesias arise is as a side-effect of dopamine replacement therapy for parkinsonism or other basal ganglia-related movement disorders. Dyskinesia can be seen either when the patient is undergoing dopamine-replacement therapy (in the case of chorea and/or dystonia) or even when off therapy (when dystonia is prevalent). Ultimately, these side-effects severely limit the usefulness of dopaminergic treatments.

Another problem associated with dopamine-replacement agents (e.g, L-DOPA and dopamine receptor agonists) is the "wearing-off" of the anti-parkinsonian efficacy of the treatment.

Another common cause of dyskinesias is the treatment of psychosis with neuroleptic drugs - this is known as tardive dyskinesia.

Dyskinesia also occurs in many other conditions including:
- Huntington's disease
- idiopathic dystonia
- Tourette syndrome
- "off" dystonia in parkinsonism
- ballism
- senile chorea

Knowledge of the pathophysiological mechanisms that underlie these disorders makes indicate that similar mechanisms mediate disorders characterized by either hyperkinesias or dyskinesias. It is to be expected, therefore, that treatments that are effective in one form of dyskinesia will be beneficial in dyskinesias of different aetiology.

Many attempts have been made to develop agents that will prevent the development of, and/or treat, dyskinesias although such attempts have met with limited success. There is, therefore, a need to develop ways by which dyskinesias may be treated.

According to a first aspect of the present invention there is provided a use of a compound of general formula I: 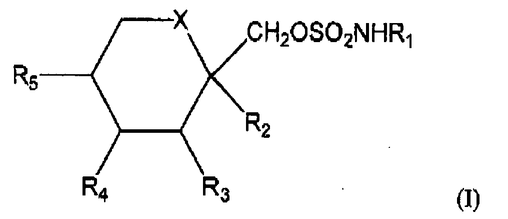 wherein X is O or CH₂;
R₂, R₃ , R₄ and R₅ are independently H, lower alkyl and R₂ and R₃ and/or R₄ and R₅ together may be a group of the following formula (II): 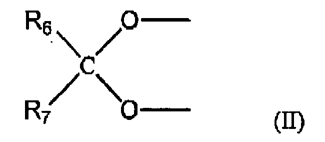 wherein R₆ and R₇ are the same or different and are H, lower alkyl or are alkyl and are joined to form a cyclopentyl or cyclohexyl ring;
in the manufacture of a medicament for the treatment of dyskinesia.

According to second aspect of the present invention there is provided a method of treating dyskinesia in a subject which comprises administering to the subject a therapeutically effective amount of a compound of general formula I.

By "dyskinesia" we mean abnormal involuntary movements that are associated with disorders of brain regions known as the basal ganglia. The dyskinesia may be a "levodopa-induced dyskinesia" that arises is a complication of the treatment of Parkinson's disease (the most common basal ganglia disease). Dyskinesia can physically manifest in two forms, chorea and dystonia. Chorea consists of involuntary, continuous, purposeless, abrupt, rapid, brief, unsustained and irregular movements that flow from one part of the body to another. Dystonia refers to sustained muscle contractions that cause twisting and repetitive movements or abnormal postures.

Dyskinesias may be distinguished from ataxia or catalepsy. Ataxia is usually associated with disorders of a part of the brain called the cerebellum, or its connections. It is characterised by poor motor coordination. There is a staggering gait (walk) and slurred speech, which may make the person appear "drunk". Catalepsy is, again, a different condition that is impossible to confuse with dyskinesias. It is usually associated with psychotic disorders. It is characterized by inactivity, decreased responsiveness to stimuli, and a tendency to maintain an immobile posture. The limbs tend to remain in whatever position they are placed. Thus, the terms dyskinesia (including chorea and dystonia), ataxia and catalepsy refer to distinct and separate disorders. They have different physical manifestations and different causes.

The present invention is based upon research conducted by the inventors relating to the activity of anticonvulsant sulphamates of general formula I. To their surprise they found that such compounds have efficacy for reducing dyskinesias.

Preferred compounds of general formula I are disclosed in detail in US 4,582,916, US 4,5123,006, US 6,420.369, US 6,559,293, US 6,583,172 and EP-B-0,138,441. The compounds disclosed in these documents may be used according to the present invention and are incorporated herein by reference. Accordingly preferred compounds that may be used according to the invention include: (tetrahydro-2H-pyran-2-yl)methane sulphamate; 2,3:4,5-bis-O-(1-methylethyidene)-β-D-fructopyranose sulphamate (see below); or 2,3:4,5-bis-O-(1-methylethyidene)-β-D-fructopyranose methylsyulphamate.

A most preferred compound is Topiramate or a functional analogue or derivative thereof.

Topiramate is a sulfamate-substituted monosaccharide that is intended for use as an antiepileptic drug. Topiramate is designated chemically as 2,3:4,5- bis-O-(1-methylethylidene)-β-D-fructopyranose sulfamate. Topiramate is sold under the registered trademark "Topamax^{®}". Topiramate has the following structural formula:

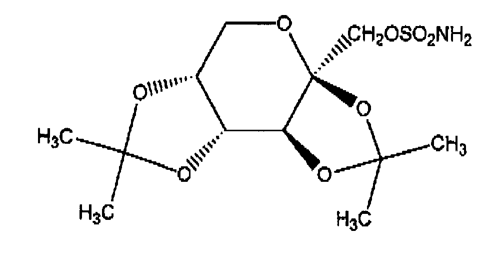

Topiramate is a white crystalline powder with a bitter taste. Topiramate is most soluble in alkaline solutions containing sodium hydroxide or sodium phosphate and having a pH of 9 to 10. It is freely soluble in acetone, chloroform, dimethylsulfoxide, and ethanol. The solubility in water is 9.8 mg/mL. Its saturated solution has a pH of 6.3. Topiramate has the molecular formula C₁₂H₂₁NO₈S and a molecular weight of 339.36.

The use of compounds according to the invention for the treatment of dyskinesias is an entirely novel invention. The basic neural mechanisms which underlie epilepsy and dyskinesias are different. One would not expect compounds such as topiramate to be effective in dyskinesias and this, therefore, represents an inventive step.

Compounds according to the invention and compositions containing such compounds may be used to treat many types of dyskinesia. For instance the compounds may be used to treat dyskinesias and hyperkinesias associated with conditions such as Huntington's disease, idiopathic torsion dystonia, tardive dyskinesia, Tourette syndrome and most particularly for dyskinesia associated with movement disorders such as parkinsonism (e.g. idiopathic Parkinson's disease, post-encephalitic parkinsonism or parkinsonism resulting from head injury), treatment of schizophrenia, drug intoxication, the effect of toxins and the like.

A clinical trial of topiramate in the condition known as essential tremor has recently been reported (Connor, GS, Neurology 2002;59:132-134) in which a mean improvement in tremor of 25% was recorded. It is important, therefore, to draw the distinction between this report and the present invention.

Firstly, essential tremor is not related to the movement disorders which are the subject of the present claims. Specifically, it is not related to Parkinson's disease, even though tremor may be present as a part of the classical triad of symptoms in the latter condition (Burne et al; J Clin Neurosci., 2002; 9: 237-242) nor is it related to conditions such as Huntington's disease, Wilson's disease, progressive supranuclear palsy (PSP), dystonia, etc.

Essential tremor differs from Parkinson's disease in many ways. Importantly, there is no known neuropathology in essential tremor, whereas the pathological basis of Parkinson's disease is well established to be degeneration of the substantia nigra, pars compacta of the midbrain. So far as they are known, the two conditions are mediated by different brain mechanisms. Furthermore, the two conditions have a different pharmacological profile. For example, L-DOPA, which is used to treat Parkinson's disease is of no value in treating essential tremor. On the other hand, essential tremor often responds to low doses of alcohol, which Parkinson's disease does not.

Secondly, the present invention relates not to the treatment of Parkinson's disease itself but dyskinesias which develop as a complication of long-term conventional treatment with L-DOPA or dopamine agonists. There is no evidence that essential tremor and dyskinesias are in any way related in terms of their cause, the brain pathways involved or their clinical pharmacological profile.

Compounds according to the invention are useful for treatment of dyskinesias which arise as a side-effect of other therapeutic agents. For instance, topiramate is useful for the treatment of dyskinesia associated with L-DOPA treatment of parkinsonism or Parkinson's disease.

Levodopa is an aromatic amino acid. The chemical name of levodopa or L-DOPA is (-)-L-α-amino-β-(3,4-dihydroxybenzene) propanoic acid. L-DOPA has the molecular formula C₉H₁₁NO₄ and a molecular weight of 197.2. Chemically, levodopa is (-)-3-(3,4-dihydroxy-phenyl)-L-alanine. It is a colorless, crystalline compound, slightly soluble in water and insoluble in alcohol. L-DOPA has the following structural formula:

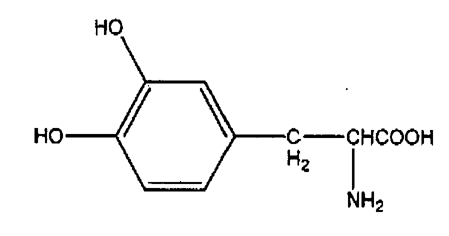

Because L-DOPA is an amino acid, it is commonly administered to patients in combination with carbidopa for the treatment of Parkinson's disease and syndrome. The chemical name for carbidopa is (-)-L-α-hydrazino-α-methyl-β-(3,4-dihydroxybenzene) propanoic acid monohydrate. Carbidopa has the empirical formula C₁₀H₁₄N₂O₄-H₂O and a molecular weight of 244.3. Anhydrous carbidopa has a molecular weight of 226.3. Sinemet^{®} is a combination of carbidopa and levodopa for the treatment of Parkinson's disease and syndrome. Sinemet^{®} is described in U.S. Patents 4,832,957 and 4,900,755, the contents of which are herein incorporated by reference. The structural formula of carbidopa is:

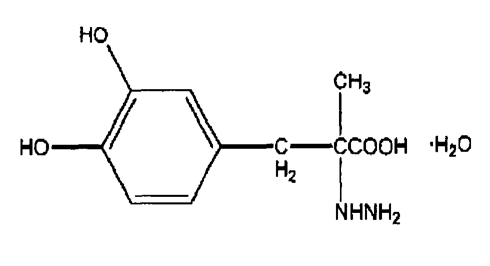

In addition, the compounds according to the invention are useful for the treatment of dyskinesias associated with ropinirole treatment. Ropinirole is a non-ergoline dopamine agonist sold under the trademark Requip^{®}. Ropinirole is the hydrochloride salt of 4-[2-(dipropylamino)ethyl]-1,3-dihydro-2H-indol-2-one monohydrochloride and has an empirical formula of C₁₆H₂₄N₂O·HCl. The molecular weight of ropinirole is 296.84 (260.38 as the free base). Ropinirole is described in U.S. Patent Numbers 4,452,808 and 4,824,860, the contents of which are hereby incorporated by reference. The structural formula of ropinirole is:

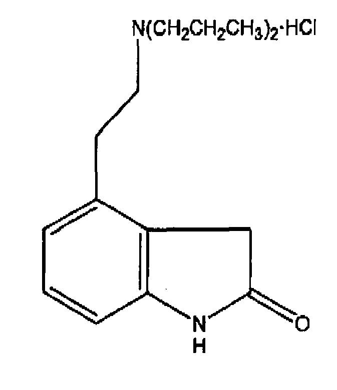

The compounds according to the invention are also useful for the treatment of dyskinesias associated with pramipexole treatment. The chemical name of pramipexole is (S)-2-amino- 4,5,6,7-tetra-hydro-6-(propylamino) benzothiazole dihydrochloride monohydrate. Pramipexole dihydrochloride is sold under the trademark Mirapex^{®}. Pramipexole dihydrochloride has the empirical formula C₁₀H₁₇N₃S-2HCl·H₂O and a molecular weight of 302.27. The synthesis of pramipexole is described in U.S. Patents 4,843,086 and 4,886,812, the contents of which are herein incorporated by reference. The structural formula of pramipexole dihydrochloride is:

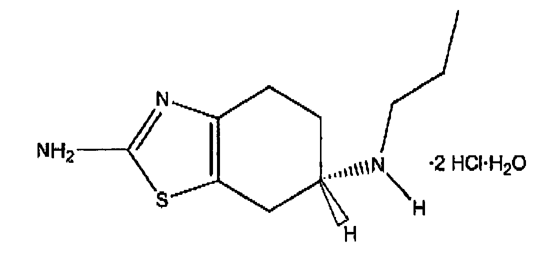

The compounds may also be used for the treatment of dyskinesias associated with cabergoline treatment. The chemical name for cabergoline is 1-adamantanamine, hydrochloride. It has a molecular weight of 187.71 and a molecular formula of C₁₀H₁₈NCl. The structural formula of cabergoline is:

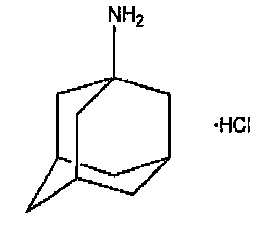

The compounds may also be used for the treatment of dyskinesias associated with bromocriptine treatment. Bromocriptine mesylate is sold under the trademark Parlodel®. The chemical name for bromocriptine mesylate is Ergotaman-3',6',18-trione, 2-bromo-12'-hydroxy-2'-(1-methylethyl)-5'-(2-methylpropyl)-, (5'a)-monomethanesulfonate. The molecular weight of bromocriptine mesylate is 750.70 and it has an empirical formula of C₃₂H₄₀BrN₅O₅· CH₄SO₃. The structural formula of bromocriptine mesylate is:

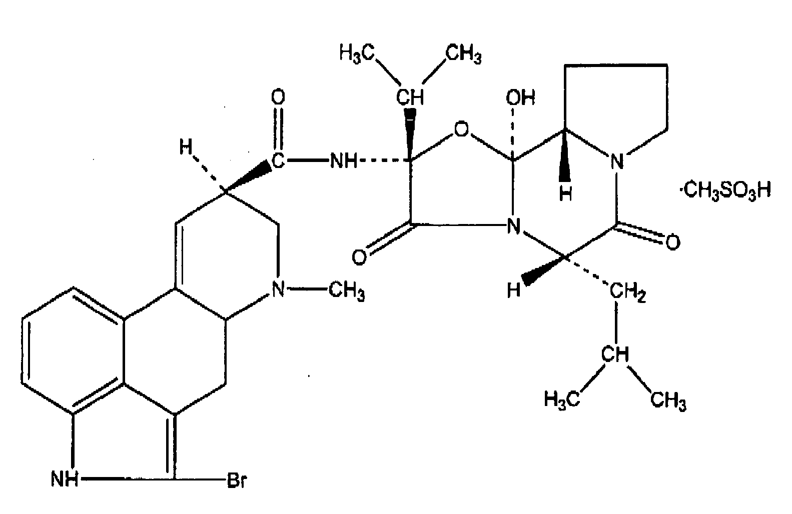

The compounds may also be used for the treatment of dyskinesias associated with lisuride treatment. The chemical name for lisuride is R(+)-N'-[(8α)-9,10-Didehydro-6-methylergolin-8-yl]-N,N-diethylurea hydrogen maleate. Lisuride has a molecular weight of 338.45 and the empirical formula C₂₀H₂₆N₄O. The structural formula of lisuride is:

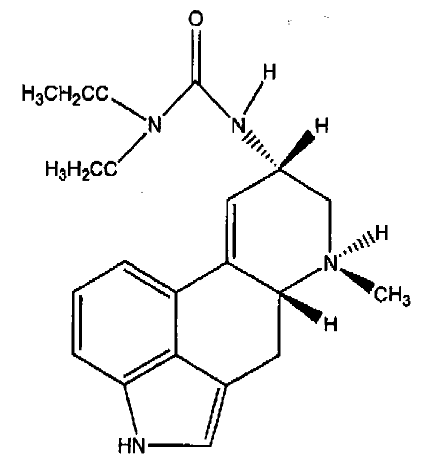

The compounds may also be used for the treatment of dyskinesias associated with pergolide treatment. The chemical name of pergolide mesylate is 8β-[(Methylthio)methyl]-6-propylergoline monomethanesulfonate. Pergolide mesylate is sold under the trademark Permax^{®}. Permax has the empirical formula C₁₉H₂₆N₂S•CH₄O₃S and a molecular weight of 410.59. The synthesis of pergolide mesylate is described in U.S. Patent Numbers 4,797,405 and 5,114,948, the contents of which are herein incorporated by reference. The structural formula of pergolide mesylate is:

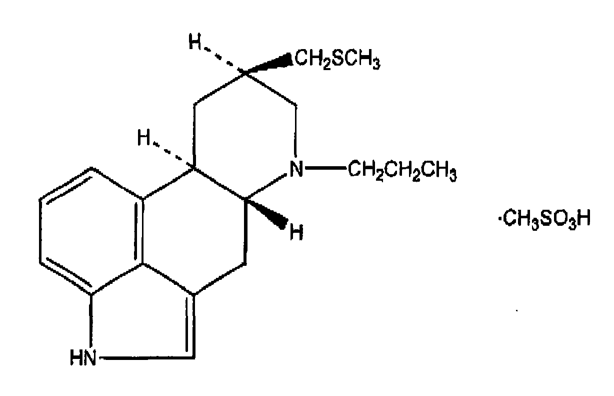

The compounds may also be used for the treatment of dyskinesias associated with apomorphine treatment. Apomorphine has the empirical formula C₁₇H₁₇NO₂ and a molecular weight of 267.33. The structural formula of apomorphine is:

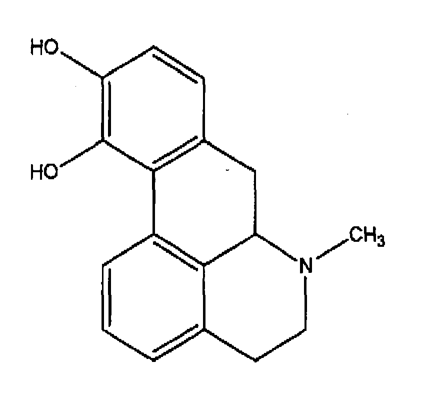

It is preferred that dyskinesias associated with the abovementioned agents are treated with topiramate. In a specific embodiment of the present invention, topiramate is used for the treatment of dyskinesia associated with L-DOPA or apomorphine treatment.

The compounds are particularly useful for treating dyskinesia caused by agents used to treat movement disorders such as parkinsonism. In this respect a use of the compositions is in the treatment of dyskinetic side-effects associated with L-DOPA or dopamine agonist therapy for parkinsonism.

The compounds may be used to treat existing dyskinesias but may also be used when prophylactic treatment is considered medically necessary, for instance, when it is considered necessary to initiate L-DOPA therapy and it is feared that dyskinesias may develop.

The compounds may be used to treat dyskinesia as a monotherapy (*i.e.,* use of the compound alone); as an adjunct to compositions to prevent dyskinetic side-effects caused by the composition (e.g. as an adjunct to L-DOPA or apomorphine given to treat parkinsonian patients) or alternatively the compounds may be given in combination with other treatments which also reduce dyskinesia (e.g. µ-opioid receptor antagonists, α₂-adrenoreceptor-antagonists, cannabinoid CB₁-antagonists, NMDA receptor-antagonists, cholinergic receptor-antagonists, histamine H3-receptor agonists, and globus pallidus/subthalamic nucleus lesion/deep brain stimulation).

The compounds may also be used as an adjunct or in combination with known therapies. For instance, we have found that the combination of L-DOPA with topiramate results in movement disorders such as Parkinson's disease being treated with significantly reduced dyskinetic side-effects.

The compounds may also be used in combination with a known neuroleptic to treat patients suffering from tardive dyskinesia. The term neuroleptic refers to the effects on cognition and behavior of antipsychotic drugs that reduce confusion, delusions, hallucinations, and psychomotor agitation in patients with psychoses. There is a naturally occurring chemical, a neurotransmitter, in the brain called dopamine. Dopamine is the chemical messenger in the brain mainly involved with thinking, emotions, behavior and perception. In some illnesses, dopamine may be overactive and upsets the normal balance of chemicals in the brain. This excess dopamine helps to produce some of the symptoms of the illness. The main effect that these drugs have is to block some dopamine receptors in the brain, reducing the effect of having too much dopamine and correcting the imbalance. This reduces the symptoms caused by having too much dopamine.

Neuroleptic drugs are a class of antipsychotics. Examples of neuroleptic compounds include: haloperidol (Haldol), chlorpromazine (Thorazine), thioridazine (Mellaril), risperidone (Risperdal), quetiapine (Seroquel), olanzapine (Zyprexa), clozapine (Clozaril), amisulpride (Solian), sertindole (Serdolect), zotepine (Zoleptil), Thiothixene (Navane), Molidone (Moban), Loxapine (Loxitane), Prochlorperazine (Compazine), Trifluoperazine (Stelazine), Perphenazine (Trilafon), and Metaclopramide (Reglan).

Haloperidol has a molecular formula of C₂₁H₂₃ClFNO₂ and a molecular weight of 375.8696 g/mol. Haloperidol is also referred to as Haldol.; 4-[4-(p-ehlorophenyl)-4-hydroxypiperidino]-4'-fluorobutyrophenone; gamma-(4-(para-Chlorophenyl)-4-hydroxypiperidino)-para'-fluorobutyrophenone; and Serenace.

Chlorpromazine hydrochloride, a phenothiazine derivative, has a chemical formula of 2-chloro-10-[3(-dimethylamino) propyl] phenothiazine monohydrochioride. Chlorpromazine hydrochloride has the molecular formula: C₁₇H₁₉ClN₂S·HCl and a molecular weight of 355.33.

SEROQUEL® (quetiapine fumarate) is an antipsychotic drug belonging to a new chemical class, the dibenzothiazepine derivatives. The chemical designation of quetiapine furnarate is 2-[2-(4-dibenzo [b,f] [1,4]thiazepin-11-yl-1-piperazinyl)ethoxy]-ethanol furnarate (2:1) (salt). Quetiapine furnarate is present in tablets as the furnarate salt. All doses and tablet strengths are expressed as milligrams of base, not as furnarate salt. Quetiapine furnarate has a molecular formula of C₄₂H₅₀N₆O₄S₂·C₄H₄O₄ and a molecular weight of 883.11 (fumarate salt).

The chemical name for clozapine is 8-chloro-11-(4-methyl-1-piperazinyl)-5H-dibenzo [b,e] [1,4] diazepine. Clozapine is a an atypical antipsychotic drug which is a tricyclic dibenzodiazepine derivative. Clozapine is sold under the trademark "CLOZARIL®". Clozapine has a molecular weight of 326.83 and a molecular formula of C₁₈H₁₉ClN₄.

The chemical name of trifluoperazine hydrochloride is 10-[3-(4-methyl-1-piperazinyl) propyl]-2-(trifluoromethyl) phenothiazine dihydrochloride. Trifluoperazine has a molecular weight of 480.43 and a molecular formula of C₂₁H₂₄F₃N₃S-2HCl.

Metoclopramide hydrochloride is a white crystalline, odorless substance, freely soluble in water. The chemical name of metoclopramide is 4-amino-5-chloto-N-[2-(diethylamino)ethyl]-2-methoxy benzamide monohydrochloride monohydrate. Metoclopramide has a molecular weight of 354.3.

Fluphenazine hydrochloride is a trifluoro-methyl phenothiazine derivative intended for the management of schizophrenia. The chemical name of fluphenazine is 4-[3-[2-(Trifluoro-methyl) phenothiazin-10-yl] propyl]-1-piperazineethanol dihydrochloride. The molecular formular of fluphenazine is C₂₂H₂₆F₃N₃OS·2HCl and its molecular weight is 510.44.

The compositions of the invention may take a number of different forms depending, in particular on the manner in which the composition is to be used. Thus, for example, the composition may be in the form of a powder, tablet, capsule, liquid, ointment, cream, gel, hydrogel, aerosol, spray, micelle, transdermal patch, liposome or any other suitable form that may be administered to a person or animal. It will be appreciated that the vehicle of the composition of the invention should be one which is well tolerated by the subject to whom it is given and enables delivery of the compounds to the brain.

The composition of the invention may be used in a number of ways. For instance, systemic administration may be required in which case the compound may be contained within a composition which may, for example, be ingested orally in the form of a tablet, capsule or liquid. Alternatively the composition may be administered by injection into the blood stream. Injections may be intravenous (bolus or infusion) or subcutaneous (bolus or infusion). The compounds may be administered by inhalation (e.g. intranasally).

The compounds may also be administered centrally by means of intracerebral, intracerebroventricular or intrathecal delivery.

The compound may also be incorporated within a slow or delayed release device. Such devices may, for example, be inserted on.or under the skin and the compound may be released over weeks or even months. Such a device may be particularly useful for patients with long-term dyskinesia such as patients on continuous L-DOPA therapy for the treatment of parkinsonism. The devices may be particularly advantageous when a compound is used which would normally require frequent administration (e.g. at least daily ingestion of a tablet or daily injection).

It will be appreciated that the amount of a compound required is determined by biological activity and bioavailability which in turn depends on the mode of administration, the physicochemical properties of the compound employed and whether the compound is being used as a monotherapy or in a combined therapy, The frequency of administration will also be influenced by the above mentioned factors and particularly the half-life of the compound within the subject being treated.

Optimal dosages to be administered may be determined by those skilled in the art, and will vary with the particular compound in use, the strength of the preparation, the mode of administration, and the advancement of the disease condition. Additional factors depending on the particular subject being treated will result in a need to adjust dosages, including subject age, weight, gender, diet, and time of administration.

Known procedures, such as those conventionally employed by the pharmaceutical industry (e.g. *in vivo* experimentation, clinical trials, etc.), may be used to establish specific formulations of compositions and precise therapeutic regimes (such as daily doses of the compounds and the frequency of administration).

Generally, a daily dose of between 0.01 µg/kg of body weight and 1.0 g/kg of body weight of a compound (e.g. topiramate) may be used for the treatment of dyskinesia depending upon which specific compound is used. More preferably, the daily dose is between 0.01 mg/kg of body weight and 100 mg/kg of body weight.

Daily doses may be given as a single administration (e.g. a daily tablet for oral consumption or as a single daily injection). Alternatively, the compound used may require administration twice or more times during a day. As an example, topiramate for treating L-DOPA induced dyskinesia in patients with Parkinson's disease may be administered as two (or more depending upon the severity of the dyskinesia) daily doses of between 25 mgs and 5000 mgs in tablet form. A patient receiving treatment may take a first dose upon waking and then a second dose in the evening (if on a two dose regime) or at 3 or 4 hourly intervals thereafter. Alternatively, a slow release device may be used to provide optimal doses to a patient without the need to administer repeated doses.

This invention further provides a pharmaceutical composition comprising a therapeutically effective amount of the compound of the invention and a pharmaceutically acceptable vehicle. In one embodiment, the amount of the compound (e.g. topiramate) is an amount from about 0.01 mg to about 800 mg. In another embodiment, the amount is from about 0.01 mg to about 500 mg. When the compound is topiramate, the amount of topiramate may be an amount from about 0.01 mg to about 250 mg; preferably about 0.1 mg to about 60 mg; and more preferably about 1 mg to about 20 mg.

In a further embodiment, the vehicle is a liquid and the composition is a solution. In another embodiment, the vehicle is a solid and the composition is a tablet. In a further embodiment, the vehicle is a gel and the composition is a suppository.

This invention provides a pharmaceutical composition made by combining a therapeutically effective amount of a compound of general formula I and a pharmaceutically acceptable vehicle.

Compounds of general formula I are preferably combined with a pharmaceutically acceptable vehicle prior to administration.

This invention provides a process for making a pharmaceutical composition comprising combining a therapeutically effective amount of a compound of general formula I and a pharmaceutically acceptable vehicle.

In the subject invention a "therapeutically effective amount" is any amount of a compound or composition which, when administered to a subject suffering from a disease against which the compounds are effective, causes reduction, remission, or regression of the disease. A "subject" is a vertebrate, mammal, domestic animal or human being.

In the practice of this invention the "pharmaceutically acceptable vehicle" is any physiological vehicle known to those of ordinary skill in the art useful in formulating pharmaceutical compositions.

In one embodiment, the pharmaceutical vehicle may be a liquid and the pharmaceutical composition would be in the form of a solution. In another embodiment, the pharmaceutically acceptable vehicle is a solid and the composition is in the form of a powder or tablet. In a further embodiment, the pharmaceutical vehicle is a gel and the composition is in the form of a suppository or cream. In a further embodiment the compound or composition may be formulated as a part of a pharmaceutically acceptable transdermal patch.

A solid vehicle can include one or more substances which may also act as flavoring agents, lubricants, solubilizers, suspending agents, fillers, glidants, compression aids, binders or tablet-disintegrating agents; it can also be an encapsulating material. In powders, the vehicle is a finely divided solid which is in admixture with the finely divided active ingredient. In tablets, the active ingredient is mixed with a vehicle having the necessary compression properties in suitable proportions and compacted in the shape and size desired. The powders and tablets preferably contain up to 99% of the active ingredient. Suitable solid vehicles include, for example, calcium phosphate, magnesium stearate, talc, sugars, lactose, dextrin, starch, gelatin, cellulose, polyvinylpyrrolidine, low melting waxes and ion exchange resins.

Liquid vehicles are used in preparing solutions, suspensions, emulsions, syrups, elixirs and pressurized compositions. The active ingredient can be dissolved or suspended in a pharmaceutically acceptable liquid vehicle such as water, an organic solvent, a mixture of both or pharmaceutically acceptable oils or fats. The liquid vehicle can contain other suitable pharmaceutical additives such as solubilizers, emulsifiers, buffers, preservatives, sweeteners, flavoring agents, suspending agents, thickening agents, colors, viscosity regulators, stabilizers or osmo-regulators. Suitable examples of liquid vehicles for oral and parenteral administration include water (partially containing additives as above, e.g. cellulose derivatives, preferably sodium carboxymethyl cellulose solution), alcohols (including monohydric alcohols and polyhydric alcohols, e.g. glycols) and their derivatives, and oils (e.g. fractionated coconut oil and arachis oil). For parenteral administration, the vehicle can also be an oily ester such as ethyl oleate and isopropyl myristate. Sterile liquid vehicles are useful in sterile liquid form compositions for parenteral administration. The liquid vehicle for pressurized compositions can be halogenated hydrocarbon or other pharmaceutically acceptable propellent.

Liquid pharmaceutical compositions which are sterile solutions or suspensions can be utilized by for example, intramuscular, intrathecal, epidural, intraperitoneal or subcutaneous injection. Sterile solutions can also be administered intravenously. The compounds may be prepared as a sterile solid composition which may be dissolved or suspended at the time of administration using sterile water, saline, or other appropriate sterile injectable medium. Vehicles are intended to include necessary and inert binders, suspending agents, lubricants, flavorants, sweeteners, preservatives, dyes, and coatings.

The compounds of general formula I can be administered orally in the form of a sterile solution or suspension containing other solutes or suspending agents (for example, enough saline or glucose to make the solution isotonic), bile salts, acacia, gelatin, sorbitan monoleate, polysorbate 80 (oleate esters of sorbitol and its anhydrides copolymerized with ethylene oxide) and the like.

A compound of general formula I can also be administered orally either in liquid or solid composition form. Compositions suitable for oral administration include solid forms, such as pills, capsules, granules, tablets, and powders, and liquid forms, such as solutions, syrups, elixirs, and suspensions. Forms useful for parenteral administration include sterile solutions, emulsions, and suspensions.

The compounds may be combined with a pharmaceutically acceptable vehicle and another therapeutically active agent prior to administration. The other therapeutically active agent may be for the treatment of parkinsonism (including Parkinson's disease).

In another embodiment of the present invention, the compound may combined with a pharmaceutically acceptable vehicle and another therapeutically active agent, wherein such agent is an antipsychotic agent used for the treatment of psychoses, prior to administration.

The invention will be illustrated further by Example and with reference to the following drawings, in which:

Figure 1 is a graphical representation of parkinsonian disability (Figure 1A) and dyskinesia (Figure 1B) timecourses following levodopa administration in MPTP-lesioned marmosets in the presence and absence of topiramate.

Figure 2 is a graphical representation of additive parkinsonian disability (Figure 2A) and dyskinesia (Figure 2B) following levodopa administration in the time period 0-1 hours post administration in MPTP-lesioned marmosets in the presence and absence of topiramate.

Figure 3 is a graphical representation of additive parkinsonian disability (Figure 3A) and dyskinesia (Figure 3B) following levodopa administration in the time period 1-2 hours post administration in MPTP-lesioned marmosets in the presence and absence of topiramate.

Figure 4 is a graphical representation of additive parkinsonian disability (Figure 4A) and dyskinesia (Figure 4B) following levodopa administration in the time period 2-3 hours post administration in MPTP-lesioned marmosets in the presence and absence of topiramate.

### EXAMPLE 1

### TOPIRAMATE REDUCES L-DOPA-INDUCED DYSKINESIA

### The MPTP-lesioned primate:

1-methyl-4-phenyl-tetxahydropyridine (MPTP), when given to non-human primates, is selectively toxic to dopamine cells and produces an animal model of Parkinson's disease. The MPTP-lesioned primate represents the best animal model of Parkinson's disease and L-DOPA-induced dyskinesia, the most common form of dyskinesia encountered in the clinical situation. The pathology, symptomatology and response of symptoms to treatments and production of side effects following treatment are very similar in the MPTP-lesioned primate to those seen in Parkinson's disease patients. Thus, when untreated, MPTP-lesioned primates show parkinsonian symptoms such as reduced range of movement, reduced speed of movement (bradykinesia) and an abnormal, hunched, parkinsonian posture. Of especial relevance to this invention is that, when treated repeatedly with L-DOPA, MPTP-lesioned primates develop the side effect of L-DOPA-induced dyskinesia in a way which is essentially indistinguishable from that seen in Parkinson's disease patients (Nash JE, et al., Exp Neurol 2000, 165:136-42; Kanda T, et al., Exp Neurol 2000, 162:321-7; Bibbiani F, et al., Neurology 2001, 57:1829-34; Konitsiotis S, et al., Neurology 2000, 54:1589-95; Blanchet PJ, et al., J Pharmacol Exp Ther 1999, 290:1034-40; Hille CJ, et al., Exp Neurol 2001, 172:189-98; Henry B, et al., Exp Neurol 2001, 171:139-46; and Fox SH, et al., Mov Disord 2001, 16:642-50).

### Preparation of MPTP-lesioned marmoset model of Parkinson's disease and L-DOPA-induced dyskinesia.

A study was performed on six adult marmosets (*Callithrix jacchus*) to assess the ability of topiramate to reduce dyskinesia, specifically L-DOPA-induced dyskinesia. The marmosets were rendered parkinsonian by subcutaneous injection of 2mg/kg MPTP for 5 consecutive days. The marmosets were allowed to recover for 18 weeks until their parkinsonism was stable. The degree of activity and disability before and after MPTP treatment were assessed using a combination of scales that measure locomotor activity, mobility, bradykinesia and posture (see below). Animals were treated with L-DOPA (12mg/kg twice daily for 6 weeks) to prime them to elicit dyskinesia. The animals had received a variety of potential anti-parkinsonian therapies prior to the current study. There was a washout period of 2 weeks between completion of any other study and the commencement of the current study.

### Details of drug administration

Topiramate or vehicle was administered orally in a volume of 5 ml/kg via a syringe in the animal's home cage. The animals were immediately transferred to an experimental cage (60cm x 55cm x 75cm, with the perch 25cm from floor of cage) for behavioral assessment.

On each day all animals also received L-DOPA (15mg/kg).

### Assessment of behavior

Behavior was assessed for 6 hours post drug administration.

A battery of behavioral tests was performed:

### 1) Dyskinesia - non-parametric measures based on the following scale:

**Dyskinesia score:** 0 = Absent, 1 = Mild, fleeting, present less than 30% of the observation period, 2 = Moderate, not interfering with normal activity, present more than 30% of the observation period, 3 = Marked, at times interfering with normal activity, present less than 70% of the observation period, 4 = Severe, continuous, replacing normal activity, present more than 70% of the observation period.

### 2) Parkinsonian disability - non-parametric measures based on the following scales:

**a) Range of movement score: 0** = no movement, 1 = movement of head on the floor of the cage, 2 = movement of limbs, but no locomotion, on the floor of the cage, 3 = movement of head or trunk on wall of cage or perch, 4 = movement of limbs, but no locomotion, on wall of cage or perch, 5 = walking around floor of cage or eating from hopper on floor, 6 = hopping on floor of cage, 7 = climbing onto wall of cage or perch, 8 = climbing up and down the walls of the cage or along perch, 9 = running, jumping, climbing between cage walls / perch / roof, uses limbs through a wide range of motion and activity. The score given was the maximum achieved in each 10 minute observation period.
**b) Bradykinesia score:** 0 = normal speed and initiation of movement, 1 = mild slowing of movement, 2 = moderate slowing, difficulty initiating and maintaining movement, marked freezing, 3 = akinetic, unable to move, with prolonged freezing episodes. The score given was representative of behaviour over the observation period.
**c) Postural abnormality score:** 0 = normal, upright, holds head up, normal balance, 1 = abnormal, crouched, face down, may lose balance. The score given was representative of behaviour over the observation period.
**d) Parkinsonian disability score:** A combination of the mobility, bradykinesia and posture scores according to the formula [18 - (Range of movement * 2) + (Bradykinesia * 3) + (Posture * 9)] to give a global parkinsonian disability rating.

Behavioral tests 1 and 2 (dyskinesia and parkinsonian disability, respectively) were assessed for 10 minutes every 30 minutes over the course of 3 hours, by *post hoc* analysis of video-recordings by an observer blinded to the treatment. The score given / achieved in each 10 minute time period was presented as defined above.

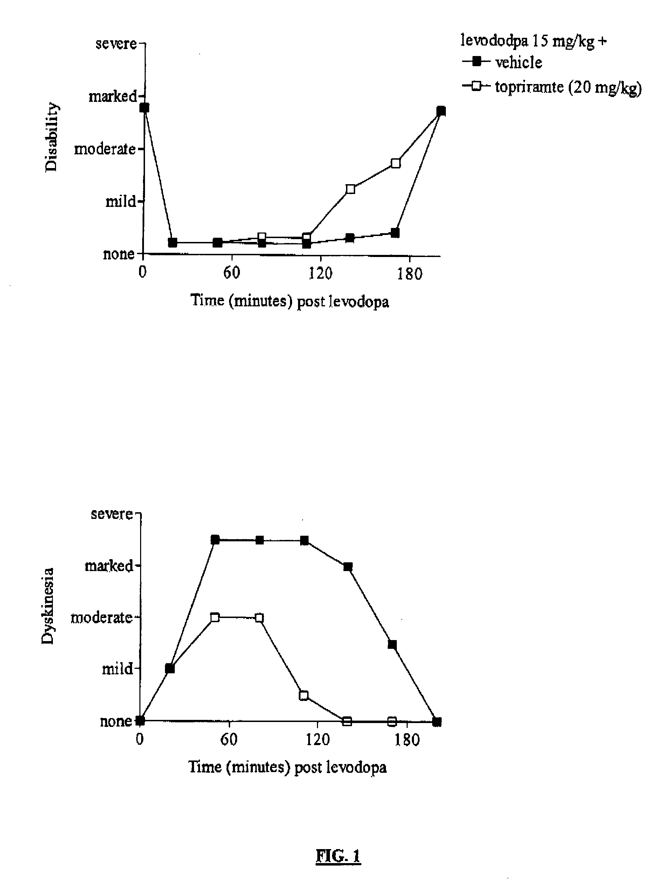

The results are shown in Figure 1A and 1B. Figure 1A: Each data point represents the median score from the group of animals (n=6). The Y-axis is labeled such that parkinsonian disability scores are presented as 0 (none), 9 (mild), 18 (moderate), 27 (marked) and 36 (severe). Figure 1B: Each data point represents the median score from the group of animals (n=6). The Y-axis is labeled such that dyskinesia scores are presented as 0 (none), 1 (mild), 2 (moderate), 3 (marked) and 4 (severe).

### Analysis of dyskinesia data

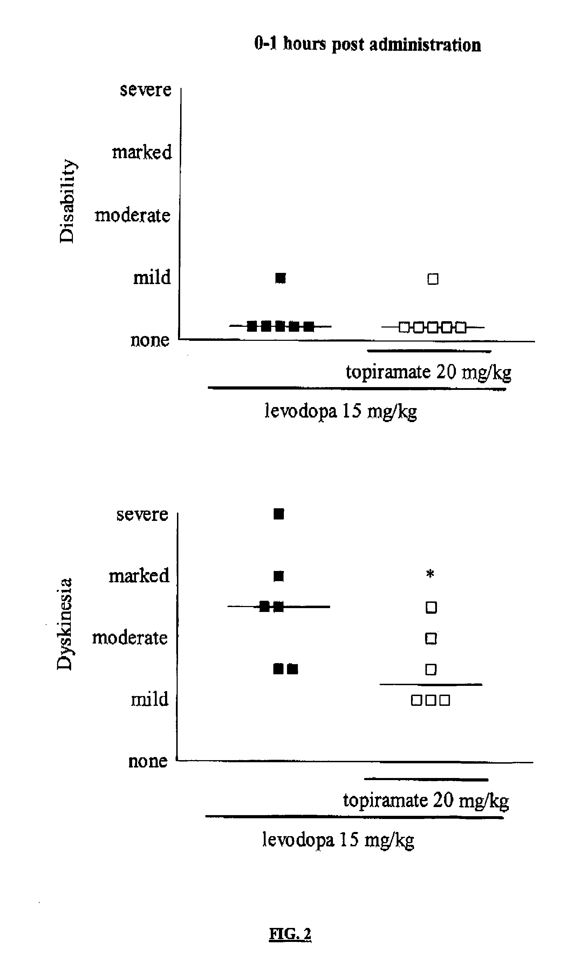

Data were collected for each one hour time period for severity of dyskinesia and were analyzed with a Wilcoxon matched pairs test (the software used was Prism version 3.0, GraphPad Software Inc). The results shown in Figures 2, 3, and 4 show the effect of topiramate treatment on MPTP-lesioned marmosets following levodopa treatment in the time period 0-1,1-2 hours, and 2-3 hours hours post administration, respectively.

The results further demonstrate that following administration of topiramate and L-DOPA, less dyskinesia was seen in comparison to L-DOPA alone.

Figure 2 shows the additive parkinsonian disability (Figure 2A) and dyskinesia (Figure 2B) following levodopa administration in the time period 0-1 hours post administration in MPTP-lesioned marmosets in the presence or absence of topiramate. Figure 2A: Data are presented as individual data from each animal, with a median for the group (horizontal line). Parkinsonian disability scores were calculated by cumulating both scores obtained in each one hour period (maximum 72). The Y-axis is labeled such that parkinsonian disability scores are presented as 0 (none), 9 (mild), 18 (moderate), 27 (marked) and 36 (severe). Data were analyzed using the Wilcoxon matched paired test, P>0.05. Figure 2B: Data are presented as individual data from each animal, with a median for the group (horizontal line). Dyskinesia scores were calculated by cumulating both scores obtained in each one hour period (maximum 72). The Y-axis is labeled such that dyskinesia scores are presented as 0 (none), 1 (mild), 2 (moderate), 3 (marked) and 4 (severe). Data were analyzed using the Wilcoxon matched paired test, *P<0.05.

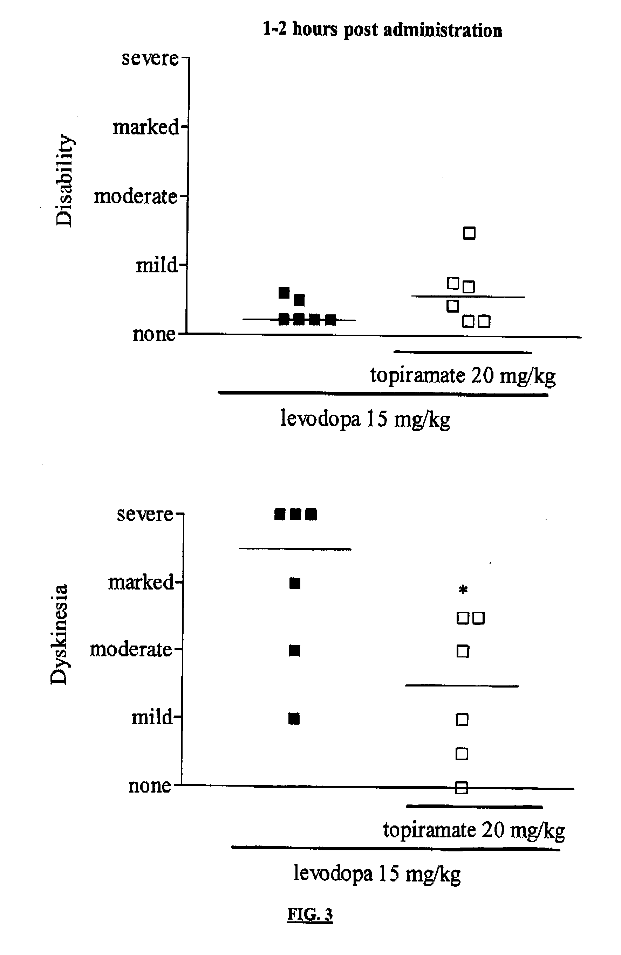

Figure 3 shows the additive parkinsonian disability (Figure 3A) and dyskinesia (Figure 3B) following levodopa administration in the time period 1-2 hours post administration in MPTP-lesioned marmosets in the presence or absence of topiramate. Details of Figures 3A and 3B are the same as Figures 2A and 2B.

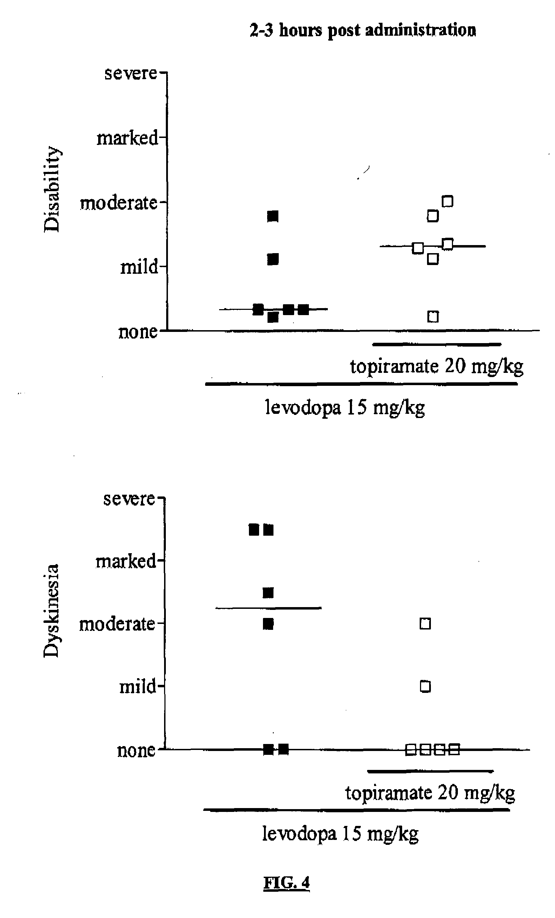

Figure 4 shows the additive parkinsonian disability (Figure 4A) and dyskinesia (Figure 4B) following levodopa administration in the time period 2-3 hours post administration in MPTP-lesioned marmosets in the presence or absence of topiramate. Details of Figures 3A and 3B are the same as Figures 3A and 3B except that in Figure 4B the analyzed data had a Wilcoxon matched paired test value of P>0.05.

The Wilcoxon matched pairs test is a nonparametric test to compare two paired groups. It is also called the Wilcoxon matched pairs signed ranks test. The Wilcoxon test analyzes only the differences between the paired measurements for each subject. The P value answers this question: If the median difference really is zero overall, what is the chance that random sampling would result in a median difference as far from zero (or more so) as observed in this experiment? If the P value is small, you can reject the idea that the difference is a coincidence, and conclude instead that the populations have different medians. This is the case in this example, P<0.05, (*i.e.*, less than a chance of 1 in 20 that the results are just coincidence).

The Wilcoxon test is the most appropriate statistical test for evaluating whether the differences between the levels of dyskinesia in the animals are different when receiving topiramate as compared to vehicle with the current study design. All animal received both topiramate and vehicle. The Wilcoxon test computes the difference between the two values in each individual animal (one with topiramate the other with vehicle) and analyzes the differences. The Wilcoxon test does not assume that those differences are sampled from a Gaussian distribution, this is important as dyskinesia is a non-parametric statistic, there is no guarantee that dyskinesia scores will be distributed in a Gaussian manner.

The results demonstrate that following administration of topiramate and L-DOPA, less dyskinesia was seen in comparison to L-DOPA alone (Figure 1B, 2B and 3B). They also demonstrate that while relieving dyskinesia, topiramate is without major effect on the anti-Parkinsonian action of L-DOPA (Figures 1A, 2A, 3A, and 4A).

One skilled in the art will readily appreciate that the specific methods and results discussed in the Example are merely illustrative of the invention as described more fully in the claims.

### REFERENCES

Bibbiani, F., et al., "Serotonin 5-HTlA agonist improves motor complications in rodent and primate parkinsonian models" Neurology 57: 1829-34 (2001).
Blanchet, PJ, et al., "Differing effects of N-methyl-D-aspartate receptor subtype selective antagonists on dyskinesias in levodopa-treated 1-methyl-4-phenyl-tetrahydropyridine monkeys" J Pharmacol Exp Ther 290: 1034-40 (1999).
Burne, et al; "The contribution of tremor studies to diagnosis of Parkinsonian and essential tremor: a statistical evaluation" J Clin Neurosci., 9(3): 237-242 (2002).
Connor, GS, "A double-blind placebo-controlled trial of topiramate treatment for essential tremor" Neurology 59(1): 132-134 (2002).
Fox, SH, et al., "Neural mechanisms underlying peak-dose dyskinesia induced by levodopa and apomorphine are distinct: evidence from the effects of the alpha(2) adrenoceptor antagonist idazoxan" Mov Disord 16: 642-50 (2001).
Henry, B., et al., "Mu- and delta-opioid receptor antagonists reduce levodopa-induced dyskinesia in the MPTP-lesioned primate model of Parkinson's disease" Exp Neurol 171: 139-46 (2001).
Hille, CJ, et al., "Antiparkinsonian action of a delta opioid agonist in rodent and primate models of Parkinson's disease" Exp Neurol 172: 189-98(2001).
Kanda T, et al., "Combined use of the adenosine A(2A) antagonist KW-6002 with L-DOPA or with selective D1 or D2 dopamine agonists increases antiparkinsonian activity but not dyskinesia in MPTP-treated monkeys" Exp. Neurol. 162: 321-7 (2000).
Konitsiotis, S., et al., "AMPA receptor blockade improves levodopa-induced dyskinesia in MPTP monkeys" Neurology 54: 1589-95 (2000).
Nash, J.E., et al., "Antiparkinsonian actions of ifenprodil in the MPTP-lesioned marmoset model of Parkinson's disease" Exp. Neurol. 165: 136-42, (2000).

## Claims

1. A use of a compound of general formula 1: 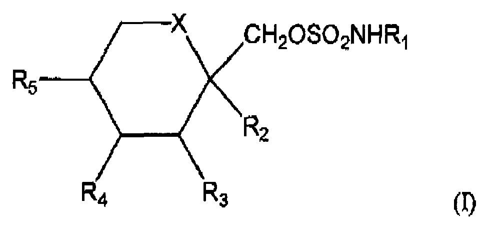 wherein X is O or CH₂;
R₂, R₃ , R₄ and R₅ are independently H, lower alkyl and R₂ and R₃ and/or R₄ and R₅ together may be a group of the following formula (II): 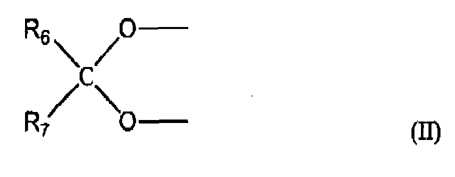 wherein R₆ and R₇ are the same or different and are H, lower alkyl or are alkyl and are joined to form a cyclopentyl or cyclohexyl ring;
in the manufacture of a medicament for the treatment of dyskinesia.

2. The use according to claim 1 wherein the compound is topiramate.

3. The use according to claim 1, or 2 wherein the dyskinesia is associated with a basal ganglia-related movement disorder.

4. The use according to claim 1 or 2, wherein the dyskinesia is associated with parkinsonism.

5. The use according to claim 4, wherein the parkinsonism is idiopathic Parkinson's disease or post-encephalitic parkinsonism.

6. The use according to claim 1 or 2, wherein the dyskinesia is associated with dopamine replacement therapy.

7. The use according to any preceding claim, wherein the dyskinesia is associated with off-dystonia in Parkinson's disease.

8. The use according to any preceding claim, wherein a therapeutically effective amount of a neuroleptic is also administered to the subject.

9. The use according to claim 8 wherein the neuroleptic is selected from the group consisting of haloperidol, chlorpromazine, quetiapine, clozapine, trifluoperazine, metoclopramide and fluphenazine.

10. The use according to claims 1 or 2, wherein the dyskinesia is associated with Huntington's disease, idiopathic torsion dystonia, tardive dyskinesia, Tourette syndrome, ballism, senile chorea.

11. The use according to claims 1 or 2, wherein the dyskinesia arises as a side-effect of a therapeutic agent.

12. The use according to claim 11, wherein the dyskinesia arises as a side-effect of the treatment of parkinsonism with a therapeutic agent.

13. The use according to claim 12, wherein the therapeutic agent is selected from the group consisting of ropinirole, pramipexole, cabergoline, bromocriptine, lisuride, pergolide, L-DOPA and apomorphine.
